# EUROPEAN PATENT SPECIFICATION

(11) **EP 3 640 230 B1**
(45) Date of publication and mention of the grant of the patent: **21.01.2026**
(21) Application number: 17913644.5
(22) Date of filing: 13.06.2017
(51) Int. Cl.: C05F 11/08, A01N 63/10, C05G 3/00, C12N 1/20

(54) **FERTILISER COMPOSITIONS AND USE THEREOF FOR IMPROVING THE SOLUBILISATION OF POTASSIUM FROM SOIL**
DÜNGERZUSAMMENSETZUNGEN UND DEREN VERWENDUNG ZUR VERBESSERUNG DER SOLUBILISIERUNG VON KALIUM AUS DEM BODEN
COMPOSITIONS FERTILISANTES ET LEUR UTILISATION POUR AMÉLIORER LA SOLUBILISATION DU POTASSIUM DANS LE SOL

(43) Date of publication of application: 22.04.2020
(73) Proprietor: Fertinagro Biotech, S.L., 44195 Teruel (ES)
(72) Inventor: ATARES REAL, Sergio, 44195 Teruel (ES); ROMERO LOPEZ, Joaquin, 44195 Teruel (ES); SALAET MADORRAN, Ignasi, 44195 Teruel (ES); FERRER GINES, Mar a, 44195 Teruel (ES); NARANJO OLIVERO, Miguel Angel, 44195 Teruel (ES); YANCE CHAVEZ, Tula del Carmen, 44195 Teruel (ES)
(74) Representative: González López-Menchero, Álvaro Luis
(86) International application number: PCT/ES2017/070427
(87) International publication number: WO 2018/229309

(56) References cited:
- WO-A1-2010/110677
- CN-A- 102 747 006
- CN-A- 104 232 538
- CN-A- 106 434 471
- US-A1- 2014 087 944
- BACHANI ET AL.: "Bioprospecting of Halotolerant Bacterial Isolates for Potassium Recovery from K-Feldspar", CHEMICAL ENGINEERING TECHNOLOGY, vol. 39, no. 9, pages 1645 - 1652, XP055554989
- SHANWARE ET AL.: "Potassium solublisers: ocurrence, mechanism and their role as competent biofertilizers", INT. J. CURR. MICROBIOL. APP. SCI., vol. 3, no. 9, 2014, pages 622 - 629, XP055554995, ISSN: 2319-7706
- AHMAD ET AL.: "Potassium-solubilizing Bacteria and Their Application in Agriculture", POTASSIUM SOLUBILIZING MICROORGANISMS FOR SUSTAINABLE AGRICULTURE, pages 293 - 313

## Description

The present invention relates to a fertiliser composition, as well as the use of said fertiliser composition to improve the solubilisation of potassium naturally present in the soil.

More specifically, the invention relates to a fertiliser composition that includes an extract of *Enterobacter xiangfangensis,* strain CECT 9264, deposited in the Spanish Type Culture Collection (CECT) with said reference, as well as niacin as a bacterial growth stimulator, in addition to a source of carbon or energy, especially carbohydrates, particularly glucose, sucrose or lactose, and/or organic acids, particularly citric acid, gluconic acid, acetic acid or combinations thereof, or a combination of carbohydrates and organic acids, as well as a solution of aminoates of cobalt, manganese, iron and zinc.

In the present invention, the term "fertiliser composition" refers to any organic or inorganic composition, natural or synthetic, both in solid and liquid state, which provides plants with one or more of the nutritional elements essential for the normal plant development thereof, usually primary macroelements (N, P, K), secondary macroelements (Ca, Mg, S) and microelements (B, Cl, Co, CU, Fe, Mn, Mo and Zn).

Thus, the term "fertiliser composition" particularly encompasses simple mineral fertilisers (contained in only one of the following macroelements: nitrogen, phosphorus or potassium) and complex mineral fertilisers (contained in more than one of the following macroelements: nitrogen, phosphorus or potassium), organic fertilisers, organominerals, etc., such as, P fertilisers, K fertilisers, N fertilisers, NP fertilisers, PK fertilisers, NK fertilisers or NPK fertilisers.

Preferably, the fertiliser composition of the invention does not include the primary macroelement K; therefore, preferably, the fertiliser composition is an N, P or NP composition.

Potassium is an essential nutrient for plant growth. Due to the large amounts of it that are absorbed by the root area in the production of most agronomic crops, it is classified as a macronutrient.

The total K content of the soil often exceeds 20,000 ppm (parts per million). Almost all of this is in the structural component of the soil minerals and is not available for plant growth. Due to the large differences in the original materials of the soil and the erosion effect of these materials, the amount of K that must be supplied to the soil varies. Therefore, the need for K in a fertilisation programme varies according to the region.

The K present in the soil can be more or less accessible to the plants depending on the state in which it is found. In this manner, K is classified into 4 interconnected groups:
1. Unavailable structural K. It is found in the crystalline structure of feldspars, clays and micas, which are part of the soil. Plants cannot use potassium in these insoluble forms. However, over time, these minerals eventually break down and small amounts of potassium are released into the soil solution.
2. Fixed or slow-exchange K. This is potassium that gradually becomes available to the plants during the growth period. Clay minerals have the ability to fix potassium. During the soil wetting and drying processes, potassium is trapped between the mineral layers (clay minerals have a layered structure). Once the soil gets wet, some of the trapped potassium ions are released into the soil solution. Slowly available potassium is not usually measured in regular soil tests.
3. Exchangeable K. It is an available source of potassium such that plants can easily absorb it. This fraction of potassium is found on the surface of clay particles and is also protected in the organic matter in the soil. It is in equilibrium with the soil solution and is easily released when plants absorb the potassium from the soil solution. Exchangeable potassium is measured in most soil tests.
4. Soluble K. It is potassium dissolved in the soil solution and is readily available for plants. It is the smallest source of potassium available.

Furthermore, the microorganisms present in the soil play a key role in the natural cycle of K and, therefore, they could provide an alternative technology to make potassium available to be absorbed by the plants. Several bacteria have been described, such as Pseudomonas, Burkholderia, Acidithiobacillus, Bacillus mucilaginosus, Bacillus edaphicus, B. circulans and Paenibacillus, which have the ability to release potassium in an manner accessible for plants starting from potassium-bearing minerals in soils (Sheng XF, 2005; Lian B., 2002; Li F.C., 2006; Liu, D., 2012). These microorganisms are capable of solubilising rock dust from potassium mineral, such as micas, illite and feldspar, through the production and excretion of organic acids (Friedrich et al, 1991; Ullman et al, 1996) or silicon ion chelates to release K in the soil solution (Friedrich et al, 1991; Ullman et al, 1996; Bennett et al, 1998). This would move potassium from groups 1 and 2 (structural and fixed) to groups 3 and 4 (interchangeable and soluble).

It has been proven that the most efficient solubilising organic acid is oxalic acid, since it forms complex structures with the aluminium ion by solubilising the silicate (Friedrich et al, 1991). One of the enzymes responsible for the synthesis of oxalic acid from oxaloacetic acid is dependent on cobalt (Hui-Q. Li, 01999). Moreover, the enzyme oxaloacetase, which also produces oxalic acid from oxaloacetic acid, requires manganese as an enhancer for the activity thereof. (A.K. Gombert, 2011).

However, the hypothesis of the organic acid production as the sole solubilisation mechanism of K is controversial. It has also been shown that the microorganism *Enterobacter xiangfangensis* accelerates the dissolution of certain silicates by producing extracellular polysaccharides (EPS) (Welch and Ullman, 1999).

Therefore, it would be desirable to have a fertiliser composition that facilitates the solubilisation of potassium with the aim of making it available in a high proportion to be absorbed by the plants.

In the article "Effect of phosphate solubilizing bacteria on the germination of cicer arietinum seeds and seedling growth", SHARMA, K et al., Journal of Herbal Medicine and Toxicology 1(1) 61-63 (2007), two phosphate-solubilising strains of Pseudomonas fluorescens and Bacillus megaterium are suggested.

The article "Bioprospecting of Halotolerant Bacterial Isolates for Potassium Recovery from K-Feldspar", BACHANI et al., Chemical Engineering Technology, 39(9) 1645-1652 discloses a composition containing Enterobacter xiangfangensis and yeast extract known to contain niacin. The effect of this bacteria as K solubilising is also acknowledged in this document.

For example, in CN106434471A, "A promoter of tobacco grown microbial compositions and uses", a microbial composition is described which includes four types of bacteria for use as a biofertiliser in tobacco cultivation, these bacteria being Bacillus (Bacillus tequilensis) CGMCC 12839, Enterobacter cloacae (Enterobacter cloacae) CGMCC 12840, Klebsiella (Klebsiella variicola) CGMCC 12845 and Enterobacter spp (Enterobacter xiangfangensis) CGMCC 12846.

With the aforementioned objective, in one aspect, the invention provides a fertiliser composition that includes an extract of *Enterobacter xiangfangensis,* strain CECT 9264, as well as niacin as a bacterial growth stimulator, in addition to a source of carbon or energy in the form of carbohydrates, particularly glucose, sucrose or lactose, and/or organic acids, particularly citric acid, gluconic acid, acetic acid or combinations thereof, or a combination of carbohydrates and organic acids, a solution of aminoates of cobalt, manganese, iron and zinc.

**The** use of the described fertiliser composition improves the solubilisation of potassium by the microorganisms present in the soil, thus facilitating the absorption of potassium by the plants.

According to an aspect, the invention provides a fertiliser composition that includes a source of carbon or energy.

In this context, the concept of "source of carbon or energy" particularly includes carbohydrates selected from the group consisting of glucose, sucrose or lactose and/or organic acids, particularly selected from citric acid, gluconic acid, acetic acid or combinations thereof, or a combination of said carbohydrates and organic acids.

In one embodiment, the source of carbon or energy is included in the composition of the invention in an amount of 30 to 50% by weight, with respect to the final weight of the composition.

As indicated above, the composition also includes a solution of aminoates of cobalt, manganese, iron and zinc as micronutrients. Preferably, this aminoate solution is present in the composition of the invention in the range of 35 to 60% by weight with respect to the total weight of the composition. In one embodiment, the minimum content of aminoates of cobalt, manganese, iron and zinc present in the aminoate solution is from 1.5 to 3% by weight, with respect to the weight of the aminoate solution.

The extract of *Enterobacter Xiangfangensis,* CECT 9264, is preferably a dehydrated extract of *Enterobacter Xiangfangensis,* being present in the composition of the invention in a range of 1 to 10% by weight with respect to the total weight of the composition.

In the present invention, "extract of Enterobacter Xiangfangensis" means the product obtained according to the following protocol:
a) The microorganism is produced by means of specific means in a bioreactor;
b) The cells of the microorganism are broken by means of the application of ultrasound or lysis means using decompression;
c) All the broth is filtered through 750 KDa tangential filters;
d) The permeate is dried by means of atomisation techniques.

The composition of the invention likewise includes niacin as a bacterial growth stimulator, preferably in a concentration of 0.1 to 200 mg of niacin per kg of composition.

According to the second aspect, the invention relates to the use of the described fertiliser composition to improve the solubilisation of potassium by the microorganisms present in the soil, thus facilitating the absorption of potassium by the plants. In one embodiment, the fertiliser composition is added to the soil in a proportion of between 5% and 20% by weight in a direct manner, either in solid or liquid form.

In order to check the effectiveness of the fertiliser composition of the invention in order to improve the solubilisation of potassium naturally present in the soil, the following tests were performed in reference to the examples.

For these tests, the mineral mica was chosen as the soil due to the high potassium content thereof, being, for example, the potassium content of the muscovite (expressed as K₂O) of approximately 10% and of the biotite of approximately 9%. Thus, this soil enables potassium solubilisation tests to be carried out with the fertiliser compositions of the invention and with comparative fertiliser compositions. Here, a mixture of soil and mica is used as soil with an insoluble potassium content (expressed as a percentage of K₂O) of 0.65%.

The comparative fertiliser composition includes the same micronutrients as the compositions of the invention, namely aminoates of cobalt, manganese, iron and zinc, and a source of carbon, in the same proportions.

### Comparative fertiliser composition:

Source of carbon or energy in the form of carbohydrates:
   40 g/100 g of total composition
Micronutrients: solution of aminoates of cobalt, manganese, iron and zinc:
   40 g/100 g of total composition.

### Composition of the invention used in the examples:

Source of carbon or energy in the form of carbohydrates:
   40 g/100 g of total composition
Micronutrients: solution of aminoates of cobalt, manganese, iron and zinc:
   40 g/100 g of total composition.
Dehydrated extract of *Enterobacter Xiangfangensis* (strain CECT 9264),
   10 g/100 g of total composition.
Niacin:
   15 mg/100 g of total composition.

In the examples, in order to confirm the solubilisation of potassium in the mica soil (soil), the different compositions of the examples and the comparative composition are applied directly and, after 12 days, the soils are analysed as far as soluble potassium present at an initial time (t=0) and after 7 days (t=7).

| | initial insoluble K₂O | final insoluble K₂O | % Solubilisation |
|---|---|---|---|
| **Example 1** | | | |
| Soil + Comparative comp. [5% (w/w)] | 0.62 | 0.62 | 0.1 |
| Soil + Comp. of the invention [5% (w/w)] | 0.62 | 0.54 | 12.6 |
| | | | |

| **Example 2** | | | |
|---|---|---|---|
| Soil + Comparative Comp. [7.5% (w/w)] | 0.62 | 0.62 | 0.0 |
| Soil + Comp. of the invention [7.5 % (w/w)] | 0.62 | 0.51 | 17.4 |
| | | | |

| **Example 3** | | | |
|---|---|---|---|
| Soil + Comparative comp. [10 % (w/w)] | 0.62 | 0.62 | 0.0 |
| Soil + Comp. of the invention [10% (w/w)] | 0.62 | 0.482 | 21.9 |

As seen in examples 1 to 3, the fertiliser composition of the invention improves the solubilisation percentage of the potassium, furthermore, in a manner dependent on the amount of composition of the invention applied to the soil.

## Claims

1. A fertiliser composition P, K, N, NP, PK, NK or NPK that includes an extract of *Enterobacter xiangfangensis,* strain CECT 9264, and niacin as a bacterial growth stimulator, the extract obtained according to the following protocol:
a) the microorganism is produced by means of specific means in a bioreactor;
b) the cells of the microorganism are broken by means of the application of ultrasound or lysis means using decompression;
c) all the broth is filtered through 750 KDa tangential filters;
d) the permeate is dried by means of atomisation techniques.

2. The fertiliser composition according to claim 1, **characterised in that** the extract of *Enterobacter xiangfangensis* is a dehydrated extract of *Enterobacter Xiangfangensis.*

3. The fertiliser composition according to claim 1 or 2, **characterised in that** the extract of *Enterobacter xiangfangensis* is present in the composition in a range of 1 to 10% by weight with respect to the total weight of the composition.

4. The fertiliser composition according to claim 1 or 2, **characterised in that** niacin is present in the composition in a range of 0.1 to 200 mg of niacin per kg of total composition.

5. The fertiliser composition according to any of claims 1 to 4, **characterised in that** it further includes a source of carbon selected from carbohydrates selected from the group consisting of glucose, sucrose or lactose, and/or organic acids, selected from citric acid, gluconic acid, acetic acid or combinations thereof, or a combination of said carbohydrates and organic acids.

6. The fertiliser composition according to claim 5, **characterised in that** the carbon source is present in the composition in a proportion of 30 to 50% by weight with respect to the final weight of the composition.

7. The fertiliser composition according to any of claims 1 to 5, **characterised in that** it also includes a solution of aminoates of cobalt, manganese, iron and zinc as micronutrients.

8. The fertiliser composition according to claim 7, **characterised in that** the solution of aminoates is present in the composition in the range of 35 to 60% by weight with respect to the total weight of the composition.

9. A use of a fertiliser composition according to claims 1 to 8 for solubilising potassium by microorganisms present in the soil, the composition being added directly to the soil, in liquid or solid form, in a proportion between 5% and 20% by weight.

## Patentansprüche

1. Düngemittelzusammensetzung P, K, N, NP, PK, NK oder NPK, die einen Extrakt aus *Enterobacter xiangfangensis,* Stamm CECT 9264, und Niacin als Stimulator für das Bakterienwachstum beinhaltet, wobei der Extrakt gemäß dem folgenden Protokoll gewonnen wird:
a) der Mikroorganismus wird mit bestimmten Mitteln in einem Bioreaktor hergestellt;
b) die Zellen des Mikroorganismus werden durch die Anwendung von Ultraschall oder Lysemitteln unter Verwendung von Dekompression aufgebrochen;
c) die gesamte Brühe wird durch 750 kDa-Tangentialfilter filtriert;
d) das Permeat wird durch Zerstäubungstechniken getrocknet.

2. Düngemittelzusammensetzung nach Anspruch 1, **dadurch gekennzeichnet, dass** der Extrakt aus *Enterobacter xiangfangensis* ein entwässerter Extrakt aus *Enterobacter xiangfangensis* ist.

3. Düngemittelzusammensetzung nach Anspruch 1 oder 2, **dadurch gekennzeichnet, dass** der Extrakt aus *Enterobacter xiangfangensis* in der Zusammensetzung in einem Bereich von 1 bis 10 Gew.-%, bezogen auf das Gesamtgewicht der Zusammensetzung, vorhanden ist.

4. Düngemittelzusammensetzung nach Anspruch 1 oder 2, **dadurch gekennzeichnet, dass** Niacin in der Zusammensetzung in einem Bereich von 0,1 bis 200 mg Niacin pro kg der Gesamtzusammensetzung vorhanden ist.

5. Düngemittelzusammensetzung nach einem der Ansprüche 1 bis 4, **dadurch gekennzeichnet, dass** sie ferner eine Kohlenstoffquelle beinhaltet, die aus Kohlenhydraten ausgewählt wird, die aus der Gruppe bestehend aus Glucose, Saccharose oder Lactose ausgewählt werden, und/oder organischen Säuren, die aus Citronensäure, Gluconsäure, Essigsäure oder Kombinationen davon ausgewählt werden, oder einer Kombination der Kohlenhydrate und organischen Säuren.

6. Düngemittelzusammensetzung nach Anspruch 5, **dadurch gekennzeichnet, dass** die Kohlenstoffquelle in der Zusammensetzung in einem Anteil von 30 bis 50 Gew.-%, bezogen auf das Endgewicht der Zusammensetzung, vorhanden ist.

7. Düngemittelzusammensetzung nach einem der Ansprüche 1 bis 5, **dadurch gekennzeichnet, dass** sie auch eine Lösung von Aminoaten von Kobalt, Mangan, Eisen und Zink als Mikronährstoffe beinhaltet.

8. Düngemittelzusammensetzung nach Anspruch 7, **dadurch gekennzeichnet, dass** die Lösung von Aminoaten in der Zusammensetzung in dem Bereich von 35 bis 60 Gew.-%, bezogen auf das Gesamtgewicht der Zusammensetzung, vorhanden ist.

9. Verwendung einer Düngemittelzusammensetzung nach den Ansprüchen 1 bis 8 zum Auflösen von Kalium durch im Boden vorhandene Mikroorganismen, wobei die Zusammensetzung in flüssiger oder fester Form in einem Anteil zwischen 5 Gew.-% und 20 Gew.-% direkt dem Boden zugesetzt wird.

## Revendications

1. Composition fertilisante P, K, N, NP, PK, NK ou NPK qui comporte un extrait d'Enterobacter *xiangfangensis,* souche CECT 9264, et de la niacine comme stimulateur de la croissance bactérienne, l'extrait obtenu selon le protocole suivant :
a) le micro-organisme est produit au moyen de moyens spécifiques dans un bioréacteur ;
b) les cellules du micro-organisme sont brisées au moyen de l'application d'ultrasons ou de moyens de lyse à l'aide de décompression ;
c) tout le bouillon est filtré à travers des filtres tangentiels de 750 KDa ;
d) le perméat est séché au moyen de techniques d'atomisation.

2. Composition fertilisante selon la revendication 1, **caractérisée en ce que** l'extrait d'*Enterobacter xiangfangensis* est un extrait déshydraté d'*Enterobacter xiangfangensis.*

3. Composition fertilisante selon la revendication 1 ou 2, **caractérisée en ce que** l'extrait d'*Enterobacter xiangfangensis* est présent dans la composition dans une plage comprise allant de 1 à 10 % en poids par rapport au poids total de la composition.

4. Composition fertilisante selon la revendication 1 ou 2, **caractérisée en ce que** la niacine est présente dans la composition dans une plage allant de 0,1 à 200 mg de niacine par kg de composition totale.

5. Composition fertilisante selon l'une quelconque des revendications 1 à 4, **caractérisée par le fait qu'**elle comporte en outre une source de carbone sélectionnée parmi les hydrates de carbone choisis dans le groupe constitué par le glucose, le saccharose ou le lactose, et/ou des acides organiques, choisi parmi l'acide citrique, l'acide gluconique, l'acide acétique ou des combinaisons de ceux-ci, ou une combinaison desdits hydrates de carbone et acides organiques.

6. Composition fertilisante selon la revendication 5, **caractérisée en ce que** la source de carbone est présente dans la composition dans une proportion de 30 à 50 % en poids par rapport au poids final de la composition.

7. Composition fertilisante selon l'une quelconque des revendications 1 à 5, **caractérisée en ce qu'**il comporte également une solution d'aminoates de cobalt, de manganèse, de fer et de zinc en tant que micronutriments.

8. Composition fertilisante selon la revendication 7, **caractérisée en ce que** la solution d'aminoates est présente dans la composition dans la plage allant de 35 à 60 % en poids par rapport au poids total de la composition.

9. Utilisation d'une composition fertilisante selon les revendications 1 à 8 pour la solubilisation du potassium par les micro-organismes présents dans le sol, la composition étant ajoutée directement au sol, sous forme liquide ou solide, dans une proportion comprise entre 5 % et 20 % en poids.
